# EUROPÄISCHE PATENTSCHRIFT

(11) **EP 3 694 598 B1**
(45) Veröffentlichungstag und Bekanntmachung des Hinweises auf die Patenterteilung: **18.08.2021**
(21) Anmeldenummer: 17832964.5
(22) Anmeldetag: 22.12.2017
(51) Int. Cl.: A61N 1/05, A61N 1/375

(54) **SYSTEM ZUR NEUROMODULATION**
SYSTEM FOR NEUROMODULATION
SYSTÈME DE NEUROMODULATION

(43) Veröffentlichungstag der Anmeldung: 19.08.2020
(73) Patentinhaber: Possover, Marc, 6032 Hagendorn (CH)
(72) Erfinder: Possover, Marc, 6032 Hagendorn (CH)
(74) Vertreter: Patent- und Rechtsanwälte Behrmann Wagner PartG mbB
(86) Internationale Anmeldenummer: PCT/EP2017/084436
(87) Internationale Veröffentlichungsnummer: WO 2019/120568

(56) Entgegenhaltungen:
- WO-A1-2017/044904
- WO-A2-2008/021524
- US-A1- 2010 312 320
- US-A1- 2014 228 905

## Beschreibung

Die vorliegende Erfindung betrifft ein System zur Neuromodulation nach dem Oberbegriff des Hauptanspruchs.

Ein derartiges System ist etwa aus der EP 2 389 975 B1 des Anmelders bekannt und beschreibt insbesondere auch speziell an die Implantationsbedingungen in einem Beckenbodenbereich des menschlichen Körpers vorteilhaft angepasste Drahtelektrodenmittel, mit welchen in gattungsgemäßer Weise die Stimulation und Neuromodulation der entsprechend kontaktierten Nerven, insbesondere Nervenwurzeln, ermöglicht ist.

Die EP 2 767 306 A1 des Anmelders offenbart im vorliegenden Anwendungskontext der Implantation von Systemen zur Neuromodulation von Nerven neben einem den Implantationsvorgang vereinfachenden Implantationswerkzeug auch weitere bevorzugte Weiterbildungen und Ausgestaltungen insbesondere der gattungsgemäßen mehrkanaligen Drahtelektrodenmittel, nämlich dergestalt, dass diese, insbesondere am eingriffseitigen (d.h. am Implantationsort einem entsprechenden Nerv zugeordneten) Elektrodenende Fixier- bzw. Ankermittel aufweisen, welche ein unbeabsichtigtes Zurückziehen oder andere, einen Eingriffszustand am Implantationsort lösende Bewegungsvorgänge verhindern bzw. unterbinden - im Fall der zum Stand der Technik zitierten EP 2 767 306 handelt es sich um Widerhaken-ähnliche Ankerstrukturen, welche die an den Implantationsort geführten Drahtelektrodenmittel durch Zusammenwirken mit einem umgebenden Muskel- bzw. Bindegewebe verankern.

Die WO 2017/044904 A1 offenbart ein Verfahren zur Behandlung eines Patienten, umfassend das Bereitstellen einer medizinischen Vorrichtung, die ein externes System und ein implantierbares System umfasst, das Implantieren des implantierbaren Systems und das Zuführen von mindestens einer Energie oder Daten an das implantierbare System mit dem externen System. Das externe System umfasst: mindestens eine externe Antenne, die konfiguriert ist, um ein erstes Übertragungssignal an das implantierbare System zu senden; einen externen Sender, der konfiguriert ist, um die mindestens eine externe Antenne zu betreiben; eine externe Stromversorgung und eine externe Steuerung. Das implantierbare System umfasst: mindestens eine implantierbare Antenne, die konfiguriert ist, um das erste Übertragungssignal von der ersten externen Vorrichtung zu empfangen; einen implantierbaren Empfänger; mindestens ein implantierbares Funktionselement, das konfiguriert ist, um mit dem Patienten zu kommunizieren; eine implantierbare Steuerung; eine implantierbare Energiespeicheranordnung; und ein implantierbares Gehäuse, das mindestens die implantierbare Steuerung und den implantierbaren Empfänger umgibt. Medizinische Geräte sind ebenfalls vorhanden.

Die US 2010/312320 A1 beschreibt eine extravaskuläre Nervenmanschette, die konfiguriert ist, um einen bleifreien, integrierten, implantierbaren Mikrostimulator aufzunehmen. Die Nervenmanschette kann einen Manschettenkörper mit einer Tasche oder einem Beutel zur abnehmbaren Aufnahme der implantierbaren Vorrichtung beinhalten. Die Nervenmanschette kann um den Nerv herum so befestigt werden, dass die Elektroden der Vorrichtung relativ zum Nerv stabil positioniert sind. Darüber hinaus treibt die Nervenmanschette den größten Teil des Stroms aus dem Stimulationsgerät in den Nerv, während sie das umgebende Gewebe vor unerwünschter Stimulation schützt.

Außerdem ist aus der US 2014/228905 A1 ein Verfahren zur Behandlung eines Patienten bekannt, welches die nachfolgenden Verfahrensschritte umfasst: Erfassen eines biologischen Parameters, der die Atmung anzeigt; Analysieren des biologischen Parameters, um einen Atemzyklus zu identifizieren; Identifizieren einer Inspirationsphase des Atemzyklus; und Liefern einer Stimulation an einen Unterzungennerv des Patienten, wobei die Stimulation durchgeführt wird, wenn eine Dauer der Inspirationsphase des Atemzyklus größer als ein vorbestimmter Teil einer Dauer des gesamten Atemzyklus ist.

Während die exemplarisch zum Stand der Technik zitierten Druckschriften, welche insbesondere auch signifikante, durch den Anmelder erreichte und aus der Implantationspraxis entwickelte Fortschritte dokumentieren, als generische Technologie zu verstehen sind und, auf der Basis üblicher Herzschrittmacher-Signalgeber als Signalerzeugungsmittel, verbunden mit mehrkanaligen Drahtelektroden, für eine Vielzahl von Stimulationsaufgaben und Indikation geeignet sind - der Anmelder steht hier für eine Vielzahl von vorteilhaft durch die insbesondere als sog. LION-Operationsmethode erreichten medizinischen Vorteile - ergibt sich gleichwohl weiterer Optimierungs- bzw. Verbesserungsbedarf im Hinblick auf die zunehmende Spezialisierung von Implantationserfordernissen und eine damit wünschenswerte Konfigurierbarkeit eingesetzter gattungsgemäßer Systeme zur Neuromodulation von Nerven.

So ist es üblich und insoweit als bekannt vorauszusetzen, ein- oder mehrkanalige Herzschrittmacher-Signalgeneratoren als gattungsgemäße Signalerzeugungsmittel mit den beschriebenen mehrkanaligen (und insoweit eine Mehrzahl von eingriffseitigen Mantelsegmentelektroden ausbildenden) Drahtelektrodenmitteln geeignet zu verbinden, diese, etwa zum Ermöglichen einer einfachen Explantation zu Wartungs- oder Austauschzwecken des Signalgenerators, im Bauchbereich vorzusehen, während dann, ermöglicht durch die Operationstechniken nach dem LION-Verfahren, die Drahtelektroden an den Einsatzbereich, etwa den inneren Beckenboden, geführt und dort gehalten werden. Weder berücksichtigt jedoch diese generische Technologie den Umstand, dass, abhängig von einer jeweiligen Indikation und einem entsprechenden Einsatz (Verwendung) des implantierten bzw. zu implantierenden Systems, einfachere oder komplexere Signalmuster zu erzeugen sind (und entsprechend die Anforderungen an die Signalerzeugerelektronik unterschiedlich sein können), noch etwa verschiedene, auch mehrfache Stimulations- bzw. Neuromodulationszwecke im Körper erforderlich sein können; in einem solchen Fall würde etwa eine Mehrzahl gattungsgemäßer Systeme mit jeweils zugeordneter, spezifizierter Operationstechnik und Indikation implantiert.

Darüber hinaus beeinflusst die Varianz der möglichen Einsatzzwecke und Modulations- bzw. Stimulationsaufgaben im menschlichen Körper, insbesondere im Beckenbodenbereich, auch den damit verbundenen unterschiedlichen elektrischen Energieversorgungsaufwand. Während entweder (zu ersetzende) Batterien oder auch (von extrakorporal) aufladbare Stromversorgungseinheiten in bekannten Signalerzeugergehäusen vorhanden sind, ist gerade deren elektrische Kapazität bzw. eine Notwendigkeit zum (Wieder-)Aufladen bzw. zum Austausch (in diesem Fall dann verbunden mit der zusätzlichen Belastung durch eine erneute operative Körperöffnung) im Regelfall nicht spezifisch angepasst und optimiert, sodass es in der Praxis zu häufig unnötigen Operationsvorgängen kommt, im Extremfall sogar zu elektrischer Energie-Unterversorgung des bereits implantierten Patienten, wiederum mit den damit verbundenen Nachteilen und Risiken, insbesondere bei kritischen Modulations- bzw. Stimulationsvorgängen zur Aufrechterhaltung von wichtigen Körperfunktionen.

Darüber hinaus stellt sich dann häufig das Problem, dass ein implantiertes Signalerzeugergehäuse - in der beschriebenen Weise üblicherweise im Bauchdeckenbereich des menschlichen Körpers implantiert - gerade bei langandauerndem Gebrauch und einem dann erforderlichen externen Zugriff (etwa zum Zweck des Austausches) erst mühsam lokalisiert werden muss, dies wiederum ist verbunden mit Problemen, von extrakorporal eine genaue Ausrichtung bzw. Position des Signalerzeugergehäuses im Körper festzustellen, was sich entsprechend nachteilig auf eine (gewünschte) kurze Zugriffs- und Explantationszeit auswirkt.

Vor dem Hintergrund dieser aus dem praktischen Implantationskontext entstehenden Probleme ist es daher Aufgabe der vorliegenden Erfindung, ein in einen menschlichen Körper implantierbares System zur Neuromodulation von Nerven nach dem Oberbegriff des Hauptanspruchs zu schaffen, welches sowohl im Hinblick auf die elektronischen Signalerzeugermittel, als auch die damit verbundenen und zusammen zu implantierenden Drahtelektrodenmittel als System und im Hinblick auf spezielle bzw. zu spezifizierende Indikationen optimiert ist. Dabei soll insbesondere ein späterer Zugriff auf bzw. eine Lokalisierung des Signalerzeugergehäuses der Signalerzeugungsmittel vereinfacht sein, und entsprechend soll damit die Voraussetzung geschaffen werden, die am Signalerzeugergehäuse ansitzenden bzw. aus diesen die Modulations- bzw. Stimulationssignale zum Nerv am Einsatzort übertragenden Drahtelektrodenmittel zuverlässig zu halten und damit auch deren Betriebssicherheit zu gewährleisten. Zusätzlich soll die Implantation eines gattungsgemäßen Systems verbessert und, insbesondere im Hinblick auf eine zielgenaue Positionierung der Drahtelektrodenmittel samt Nervenkontaktbereich am betreffenden Nerv, vereinfacht werden.

Die Aufgabe wird durch das implantierbare System zur Neuromodulation nach dem Hauptanspruch gelöst; vorteilhafte Weiterbildungen der Erfindung sind in den Unteransprüchen beschrieben.

In erfindungsgemäß vorteilhafter Weise weist zunächst das Signalerzeugergehäuse einen flanschartig und/oder als Gewebestück ausgebildeten, am Gehäuse ansitzenden und/oder vorspringenden und flachen Befestigungsabschnitt auf, mit welchem es einem Operateur, der das erfindungsgemäße System im Körper implantiert, ermöglicht ist, dieses Signalerzeugergehäuse durch operative Verfahren an einem Knochenbereich im Körper festzulegen.

Dabei hat es sich als besonders bevorzugt herausgestellt, als einen derartigen Knochenbereich einen Bereich des Schambeins auszuwählen, da auf diese Weise nicht nur zur Lösung der gestellten Aufgabe und in erfindungsgemäß vorteilhafter Weise eine sichere, einfach zugreifbare und für einen am Signalerzeugergehäuse angekoppelten Elektrodendraht (für Beckenboden-bezogene Indikationen) günstige Implantationsposition gefunden ist, auch ermöglicht es gerade die Implantation und Fixation im Bereich des Schambeins, erfindungsgemäß vorteilhaft realisiert durch den flachen Befestigungsabschnitt, von extrakorporal und durch die das Schambein bedeckende Haut- bzw. Muskelschicht, verschiedenste Signal zuzuführen, mit welchen sich dann nicht nur das sicher im Körper fixierte Gehäuse z.B. von extrakorporal mit elektrischer Ladeenergie versorgen lässt, auch ist es etwa möglich (und gemäß vorteilhafter Weiterbildungen der Erfindung vorgesehen), das System mit geeignet ausgestalteten drahtlosen Steuer-, Parametrisierungs- und/oder Betriebsmodusmitteln in einen jeweils gewünschten Betriebszustand zu versetzen.

Insoweit ist die vorliegende Erfindung als mehrfach synergistisch zu verstehen, denn sie sorgt nicht nur für einen auch zukünftige (operative) Zugriffe erleichternden physisch-mechanischen Befestigungszustand des Signalerzeugergehäuses im Körper (bei damit gleichzeitig ermöglichter definierter Ausrichtung, was wiederum die Führungs- und Kontaktsicherheit des zugehörigen Elektrodendrahts (Drahtelektrodenmittel) verbessert), auch ist durch diese erreichbare und definierte Implantationsposition die Voraussetzung für die beschriebene, von extrakorporal vorzugebende Steuerung bzw. Parametrisierung verschiedenster Betriebs- und Einstellmodi gegeben.

In erfindungsgemäß vorteilhafter Weise und die Erfindung weiterbildend ist der Befestigungsabschnitt elastisch verformbar ausgebildet und weist entweder vorbestimmte bzw. vollständig oder teilweise eingeformte Durchbrüche auf (welche weiter bevorzugt in Form eines regelmäßigen Musters vorgesehen sein können), alternativ aber auch in Form eines Vlies-Polymermaterials und/oder Gewebes ausgestaltet, um damit, durch den beschriebenen chirurgischen Eingriff durch einen Operateur, die Festlegung mittels Nahtmaterial, Schrauben, Nägeln, Klammern oder anderen Techniken am Knochenbereich einfach, mit entsprechend kurzer Operationszeit, hoher Befestigungsgüte und mit geringstmöglicher Belastung für den Patienten ausführen zu können.

Dabei hat es sich in der Praxis als besonders vorteilhaft herausgestellt, den erfindungsgemäßen Befestigungsabschnitt axial beidseits (beidends) von Schmalseiten eines Signalerzeugergehäuses auszubilden, welches im Rahmen dieser Weiterbildung zusätzlich vorteilhaft langgestreckt ausgebildet sein kann. Dies ermöglicht dann etwa das Entlangführen (Ausrichten) des Gehäuses an einem schmalen Schambeinabschnitt, wobei dann die endseitigen flanschartigen Abschnitte ein besonders einfaches und sicheres mechanisches Verbinden mit dem Knochen ermöglichen.

Die durch die vorliegende Erfindung ermöglichte und vorgesehene Fixation des Signalerzeugergehäuses am Knochenbereich legt mit dieser Maßnahme gleichermaßen das Einspeisungs- bzw. Signalerzeuger-seitige Ende der Drahtelektrodenmittel fest. Zur weiteren Optimierung der Führung und Sicherung dieser Drahtelektrodenmittel sieht dann eine weitere vorteilhafte Weiterbildung der Erfindung vor, die Drahtelektrodenmittel durch Fixiermittel (weiter bevorzugt am nerven- und damit eingriffsseitigen Ende) zu sichern, welche eine mittels Nahtmaterial im implantierten Zustand zu fixierende Öse vorsehen. Diese am freien Ende des Elektrodendrahtes vorgesehene Öse gestattet insbesondere auch solchen Operateuren bzw. Chirurgen eine sehr sichere (und insbesondere dann auch gegenüber einem unbeabsichtigten Zurückziehen des Drahtes gesicherte) Verankerung der Elektrodenmittel sicherzustellen, die aus ihrer üblichen Operationspraxis schnelle und sichere Nahtmaterial-Knoten herstellen können. Insoweit erreicht damit die weiterbildungsgemäße Öse einen signifikanten Fixationsvorteil etwa gegenüber den als bekannt vorausgesetzten Widerhaken zur Elektrodenverankerung.

Wiederum die letztgenannte vorteilhafte Weiterbildung erweist sich zusätzlich dann als besonders vorteilhaft, wenn, gemäß weiterer vorteilhafter Ausgestaltung der Erfindung, das Signalerzeugergehäuse ein lösbares Befestigen (etwa durch eine geeignete Stecker-Buchsenkombination) des Elektrodendrahtes vorsieht. In einem solchen Fall ist es dann nämlich ohne Explantation des (ja bereits am Eingriffsort sitzenden und potentiell auch bereits eingewachsenen) Elektrodendrahtes ermöglicht, das Signalerzeugergehäuse auszuwechseln, möglicherweise nicht nur mit dem Zweck eines Austausches einer Spannungsversorgung, sondern etwa auch im Fall technischer Aktualisierungen, Weiterentwicklungen oder dergleichen Änderungen bei (üblicherweise langandauernden und bis zu mehrjährigen) langen Betriebs- und Tragezeiten eines implantierten Systems. Wiederum die erfindungsgemäß weiterbildend vorgesehene Lösbarkeit der Elektrode vom Signalerzeugergehäuse ermöglicht darüber hinaus dann weiterbildungsgemäß etwa auch das vorteilhafte Vorsehen einer Mehrzahl von Drahtelektrodenmitteln an einem (einzelnen) Signalerzeugergehäuse, welches dann nicht nur mehrkanalig, sondern auch zur simultanen Signalerzeugung für eine Mehrzahl von Drahtelektroden ausgebildet sein kann, die wiederum an potentiell verschiedene Implantationsorte (mit entsprechend verschiedenen Indikationen) geführt (und potentiell weiterbildend auch entsprechend mit den Ösen fixiert) sein können. Wiederum gestattet es dann der erfindungsgemäß weiterbildende Gedanke der Stecker- bzw. Buchsenverbindung, nicht nur die Lösbarkeit im Hinblick auf die Drahtelektroden vorzusehen, sondern auch potentiell damit eine elektrische Spannungs- bzw. Energieversorgung flexibel austausch- und lösbar (ggf. auch redundant bzw. durch ein zusätzlich zum Signalerzeugergehäuse zur Implantation vorgesehenes weiteres Gehäuse (Spannungsversorgungsgehäuse)) zu realisieren.

Auch im Fall dieser Weiterbildung sind die im Rahmen der Erfindung möglichen Ausgestaltungen nahezu beliebig - so ist es etwa möglich, die mittels dieser (zusätzlichen) Spannungsversorgungsmittel ermöglichte Energieversorgung der Signalerzeugungsmittel von extrakorporal aufladbar auszugestalten (etwa induktiv durch die Bauchdecke, indem das Spannungsversorgungsgehäuse entsprechend oberflächennah implantiert wird), ergänzend oder alternativ ist es gar denkbar, körpereigene Energie, nämlich die Wärmeenergie, mithilfe thermisch wirksamer Spannungserzeugungsmittel in elektrische Energie umzusetzen und so für eine potentiell von Batterien bzw. externer Energiezuführung unabhängige Energieversorgung zu sorgen. Gerade die Fortschritte in der Mikroelektronik, der energiesparenden integrierten Schaltungstechnik und einer (mit entsprechend niedrigeren Modulations- bzw. Stimulationspegeln verbundenen) präziseren Signalansteuerung verspricht eine Nutzbarkeit derartiger autarker Energiesysteme.

Im Rahmen einer weiteren bevorzugten Ausführungsform der vorliegenden Erfindung ist es zudem möglich, das erfindungsgemäße System vorkonfiguriert auf vorbestimmte Indikationen auszugestalten. Dabei sind dann die erfindungsgemäßen, im Signalerzeugergehäuse vorgesehenen Signalerzeugungsmittel zum Erzeugen eines dieser Indikation entsprechenden Signalerzeugungsmusters (Signalmusters) für die Elektrodenansteuerung konfiguriert, sodass keine spezielle weitere Programmierung erfolgen muss (ggf. kann, als mögliche Weiterbildung, durch Umschalten aus einer begrenzten Anzahl vorkonfigurierter Signalerzeugungsmuster ausgewählt werden). Entsprechend ist die (weiter bevorzugt fest und unlösbar mit dem Signalerzeugergehäuse verbundene) Drahtelektrode (Drahtelektrodenmittel) etwa im Hinblick auf eine Elektrodenlänge, und/oder eine Anzahl und/oder Anordnung von Mantelsegmentelektroden an die Indikation angepasst, sodass, mit einem Minimum an notwendiger Einricht- und Konfigurationsarbeit, ein entsprechend für die vorgesehene Indikation angepasstes System implantiert werden kann. Dieses bietet zudem den Vorteil vereinfachter Herstellbarkeit und eines minimierten Hardwareaufwandes, sodass auch in dieser Richtung Effizienz- und Kostenvorteile erreichbar sind. Wenn dann, eine Notwendigkeit besteht, an mehreren Nerven im Körperinneren zu therapieren oder zu verschiedenen Stimulationszwecken mehr als eine Elektrode zu implantieren, könnte ein derartiges, dezidiert vorkonfiguriertes und entsprechend vereinfachtes System dann auch in Mehrzahl (und jeweils vorkonfiguriert) implantiert werden. Die etwa auch mit derartigen, speziell auf gewisse Indikationen zugeschnittenen bzw. vorkonfigurierten Systeme bieten zudem den Vorteil üblicherweise geringer elektrischer Energieaufnahme, sodass zu erwarten steht, dass eine (Batterie-)Stromversorgungsquelle im Signalerzeugergehäuse über eine Gesamt-Systemlebensdauer nicht ausgetauscht (oder geladen) werden muss, wiederum mit dem Potential weiterer Kosten- und Effizienzverbesserung.

Die vorstehend beschriebenen flexiblen Möglichkeiten des Verbindens von implantierten Signalerzeugungs- und/oder Spannungsversorgungsgehäusen bieten ferner im Rahmen der Erfindung und weiterbildungsgemäß die Möglichkeit, selbst auch zwei Signalerzeugergehäuse (mit jeweils darin vorgesehenen Signalerzeugungsmitteln) geeignet drahtgebunden zu koppeln, wenn diese gemeinsam in einem menschlichen Körper implantiert sind. Eine derartige (drahtgebundene) Kopplung kann dann sowohl eine Kopplung auf Versorgungsspannungsebene bedeutet (sodass eine Versorgungsspannungsquelle eines ersten Gehäuses dann auch das zweite Gehäuse versorgen kann), ergänzend oder alternativ könnte eine Signal- und/oder Datenkommunikation stattfinden, etwa mit einem Zweck, die (mindestens zwei) Signalerzeugungsmittel geeignet zu synchronisieren, z.B. in der Absicht, therapeutisch unerwünschten gemeinsamen Betrieb auszuschließen.

Wie vorstehend bereits diskutiert, gestattet gerade die erfindungsgemäß ermöglichte Fixation des Signalerzeugergehäuses am Knochenbereich des Körpers die extrakorporale Ansteuerung durch die weiterbildungsgemäß vorgesehenen (und insbesondere auch zum mobilen bzw. portablen Betrieb, weiter bevorzugt zur manuellen Handhabung vorgesehenen) Steuer-, Parametrisierungs- bzw Betriebsmodusmittel: Mithilfe üblicher Technologien wie etwa Bluetooth oder anderer hochfrequenz-basierter (und weiter bevorzugt digitaler) Kommunikationstechniken lässt sich entsprechend eine einfache und betriebssichere Kommunikation zum implantierten Signalerzeugergehäuse herstellen, sodass dann, entweder durch einen behandelnden Arzt, ergänzend oder alternativ durch den implantierten Patienten selbst, geeignete Betriebsmodi und/oder Parametrisierungen des implantierten Systems einstell-, vorwähl- und ansteuerbar sind. Neben einem (einfachen) Ein- und Ausschalten, etwa einer zum Zweck der Sexualstimulation implantierten und mit einer entsprechend ausgerichteten Elektrode verbundenen Signalerzeugungselektronik als Beispiel, sind nahezu beliebige Konstellationen denkbar, das Verhalten des implantierten Systems zu beeinflussen. Darüber hinaus wird weiterbildungsgemäß eine derartige Kommunikation auch bidirektional ausgestaltbar sein, etwa mit dem Zweck, unmittelbare Betriebszustands-, Status- oder Warnsignale außerhalb des Körpers empfangen zu können, sodass damit dann auch eine deutlich verbesserte Qualitätskontrolle des Betriebs des implantierten Systems, bis hin zu konkreten Sicherheitsmechanismen, denkbar sind. Diesbezüglich sieht etwa eine besonders bevorzugte Weiterbildung der Erfindung vor, den erfindungsgemäßen Steuer-, Parametrisierungs- bzw. Betriebsmodusmitteln Mittel zur akustischen und/oder optischen Darstellung von verschiedensten Konfigurations-, Stromversorgungs- bzw. anderen Betriebszuständen der Signalversorgungsmittel zuzuordnen, welche dann (typischerweise in einem bidirektionalen Kommunikationsbetrieb) extrakorporal empfangen und geeignet zur Überwachung bzw. Diagnose aufbereitet werden können. Dabei dient es dann auch der Daten- und Manipulations- bzw. Zugriffssicherheit, diesen Betrieb geeignet nutzer- bzw. geräteidentifizierend auszugestalten.

Die erfindungsgemäß vorgesehenen Elektrodendrahtführungsmittel sind in der Art einer langgestreckten und bogenförmig ausgestalteten Hülse ausgebildet, welche, bevorzugt einends, einen Betätigungsabschnitt zur manuellen Führung aufweist. Diese Hülse, mit einem zusätzlich erfindungsgemäß darin vorgesehenen, am dem Handhabungsbereich (Betätigungsabschnitt) entgegengesetzten Einführungsende einen Dorn mit Eingriffsspitze für ein Durchdringen des menschlichen Körpergewebes aufweisend, ermöglicht dann, etwa aus einer rektal-extrakorporalen Richtung, das Eindringen in den menschlichen Körper bis zu einer vorgesehenen Implantationsposition, bevorzugt unter laporoskopsich-endoskopischer Kontrolle. Nach Entfernung des (von der eigentlichen Hülse lösbaren) Dorns, bevorzugt etwa durch Herausziehen entlang der Einführungsrichtung (und dann Abtransportieren etwa durch den Arbeitskanal des Endoskops), kann dann, aus der Richtung der Implantationspostition (d.h. bei bereits eingesetztem Signalerzeugergehäuse und - bevorzugt unlösbar - daran ansitzender Drahtelektrode), die Drahtelektrode (Drahtelektrodenmittel) durch die Hülse (Elektrodendrahtführungsmittel) eingeführt werden (indem sie operativ am (nunmehr offenen) Endbereich der Hülse gegriffen und dort eingeführt wird). Die Elektrode sitzt dann nach einem Herausziehen der Elektrodendrahtführungsmittel (hier entgegen der Einführungsrichtung) bereits an der korrekten Position an dem vorgesehenen Nerv. Im Rahmen bevorzugter Indikationen für die Verwendung der Erfindung eignet es sich dabei insbesondere, das System mit den Elektrodendrahtführungsmitteln zu benutzen, um entsprechend von Drahtelektrodenmittel an für die Genital- bzw. Sexualstimulation relevante Nerven (etwa den Nervus Cavernosi (Schwellkörpernerv)) bzw. an dem dem Penis bzw. der Klitoris zugeordneten Abschnitt des Rückennervs zu bringen.

Im Ergebnis ermöglicht das vorliegende und erfindungsgemäße System völlig neue und überraschende Möglichkeiten sowohl in der Implantation selbst, als auch im Betrieb des erfindungsgemäß verbesserten Systems, sodass nicht nur zur erwarten steht, dass durch die vorliegende Erfindung die Sicherheit und die Qualität der damit ermöglichten Implantationsvorgänge deutlich erhöht werden dürfte. Auch ist zu erwarten, dass über einen (selbst mehrjährigen) Betrieb des Systems im implantierten Zustand die Betriebssicherheit, der Bedienkomfort und die Sicherheit des Systems deutlich werden können.

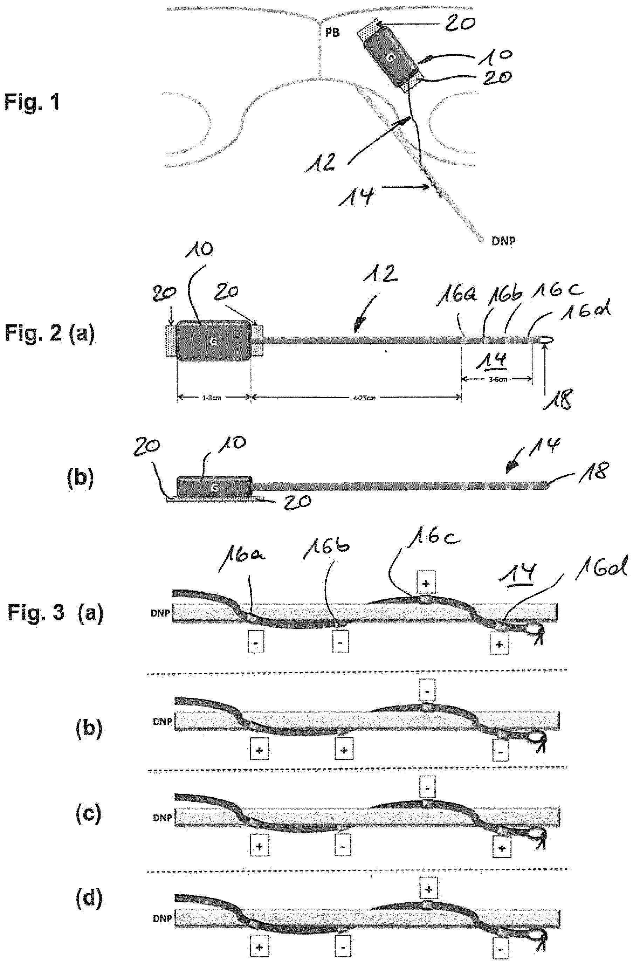

Weitere Vorteile, Merkmale und Einzelheiten der Erfindung ergeben sich aus der nachfolgenden Beschreibung bevorzugter Ausführungsbeispiele sowie anhand der Figuren; diese zeigen in
- Fig. 1: eine Schemadarstellung des erfindungsgemäßen Systems zur Neuromodulation mit einem exemplarisch am Schambein eines menschlichen Körpers befestigten Signalerzeugergehäuses sowie einer mit dem Signalerzeugergehäuse (bzw. darin aufgenommenen Signalerzeugungsmitteln) verbundenen Drahtelektrode, welche bis zu einem Abschnitt des Dorsalnervs (des betreffenden Sexualorgans, etwa des Penis oder der Klitoris) geführt ist und mit seinem Nervenkontaktbereich diesen Nervenabschnitt umwickelt;
- Fig. 2: in Teilfiguren (a) und (b) Ansichten der Kombination aus Signalerzeugergehäuse und darin ansitzenden Drahtelektrodenmitteln mit exemplarischen Maßen, insbesondere für die Indikation bzw. Verwendung im Ausführungsbeispiel der Fig. 1, wobei Teilfigur (a) eine Draufsicht und Teilfigur (b) eine Seitenansicht dieser Kombination zeigt;
- Fig. 3: mit Teilfiguren (a) bis (d) Detailansichten des Nervenkontaktabschnitts der hier vier Mantelsegmentelektroden ausbildenden Drahtelektrodenmittel, mit jeweils unterschiedlichen möglichen Signal-Ansteuerungszuständen und entsprechenden Polaritäten;

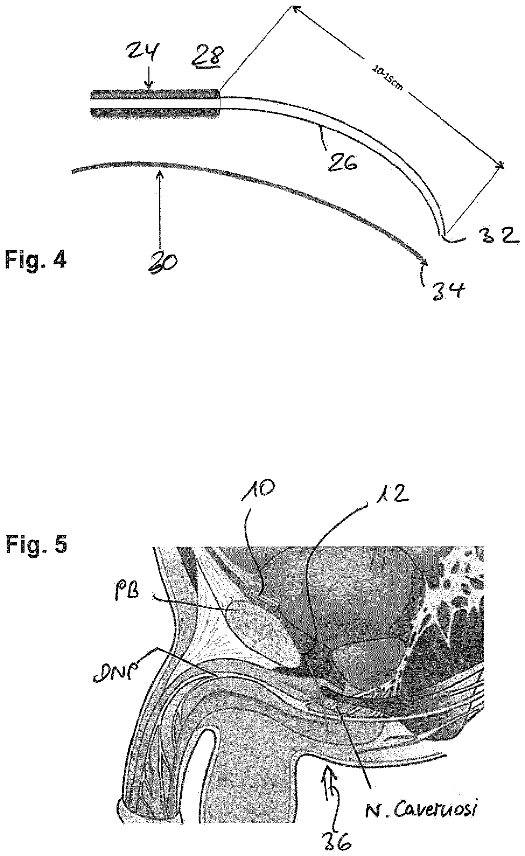

- Fig. 4: eine Schemaansicht der das System des gezeigten Ausführungsbeispiels weiterbildenden Elektrodendrahtführungshülse gemäß einem Ausführungsbeispiel mit Griff- bzw. Führungsabschnitt und zum Durchdringen von Körpergewebe ausgebildetem Spitzenabschnitt, wobei die Darstellung der Fig. 4 zusätzlich im unteren Bereich einen zum Einsetzen in diese Hülse ausgebildeten Dorn zeigt, welcher dann an der Zielposition der Führungsmittel im Körperinneren entfernbar ist;
- Fig. 5: eine anatomische Schnittansicht durch den Genitalbereich des menschlichen Körpers mit schematisch am Schambeininneren vorgesehenem Signalerzeugergehäuse und sich von diesem in den Nervenbereich erstreckenden Drahtelektrodenmitteln, welche entsprechend ihres Verlaufs durch die Führungsmittel gemäß Fig. 4 bestimmt sind.

Wie zunächst die Darstellungen der Fig. 1 und 2 verdeutlichen, weist das System zur Neuromodulation von Nerven gemäß einem ersten Ausführungsbeispiel der Erfindung ein in einem menschlichen Körper implantierbares Signalerzeugergehäuse 10 auf, aus welchem sich eine Drahtelektrode 12 erstreckt, welche wiederum an einem (dem Gehäuse 10 entgegengesetzten) Ende einen Nervenkontaktbereich 14 ausbildet. Wie diesbezüglich insbesondere die Detailansichten von Fig. 2 und Fig. 3 erkennen lassen, ist die Drahtelektrode 12 vierpolig ausgestaltet, mit entsprechend vier Mantelsegmentelektroden (16a bis 16d), welche, im gezeigten Ausführungsbeispiel, sich entlang eines Endabschnitts von ca. 3cm bis 6cm der typischerweise mit einer Länge zwischen 4cm und 31cm aus dem Gehäuse 12 herausstehenden Drahtelektrode erstrecken. Endseitig dieses Nervenkontaktbereichs ist am Drahtende noch eine Befestigungsöse 18 ausgebildet, welche, vergleiche etwa die Darstellungen der Fig. 3 (a) bis (d), mit einer Nahtmaterialverbindung am Implantationsort geeignet vom Operateur befestigt werden kann und damit das endseitige Festlegen der Drahtelektrode gestattet.

An Schmalseiten des in der Draufsicht rechteckförmigen Signalerzeugergehäuses 10 sind netz- bzw. gewebeartige und hier flexibel ausgebildete Befestigungsflansche 20 vorgesehen, welche, vgl. etwa die Montagedarstellung der Fig. 1, an einem inneren Schambeinbereich des menschlichen Schambeins (PB) durch bekannte Techniken wie etwa Nageln oder Klammern befestigt sind; in die Befestigungsflansche bereits eingebrachte Durchbrüche vereinfachen diese Befestigung.

Im gezeigten Ausführungsbeispiel ist die Drahtelektrode mit ihrem Nervenkontaktbereich 14 (bzw. den dort vorgesehenen Mantelsegmentelektroden 16a bis 16d) mit einem Abschnitt des Penis-Dorsalnervs (DNP) verbunden, wobei, wie insbesondere die Vergrößerungsdarstellungen der Fig. 3 verdeutlichen, der Elektrodendraht mit ca. 1,5 Windungen um den gezeigten Abschnitt des DNP gewunden ist; die Teilfiguren (a) bis (d) von Fig. 3 verdeutlichen mögliche Polaritäten eines (DC-)Ansteuersignals, diese sind jedoch rein exemplarisch und können auch anders, insbesondere auch als Wechselsignale, gesteuert und konfiguriert werden.

Die Fig. 4, mit Implantationsergebnis in Fig. 5, verdeutlicht eine weitere vorteilhafte Weiterbildung der Erfindung in Form eines dem System vorteilhaft zugehörigen Elektrodendraht-Führungsinstruments: Die gezeigte, einen Handhabungsabschnitt 24 sowie einen gebogenen Abschnitt 26 aufweisende Hülse 28 eignet sich nämlich, in insbesondere auch für wenig geübte Operarateure zuverlässiger Weise, den Elektrodendraht (etwa der Vorrichtung gemäß Fig. 1 bis 3) an eine gewünschte Implantationsposition zu bringen, wie es etwa die schematische Anatomiedarstellung der Fig. 5 verdeutlicht: Konkret bildet das Instrument 28, mit zunächst darin eingesetztem Dorn 30 (unterer Teil der Fig. 4), welcher eingriffs- bzw. endseitig eine aus einer Eingriffsöffnung 32 des Hülseninstruments 28 hervorstehende Spritze 34 ausbildet, die Möglichkeit, von extrakorporal, nämlich entlang Pfeilrichtung 36 in Fig. 5, diese Werkzeugkombination durch das menschliche Gewebe zu führen, bis zum Schambeinbereich PB. An diesem ist üblicherweise bereits operativ das Signalerzeugergehäuse 10, etwa entsprechend der in Fig. 1 gezeigten Art, befestigt oder liegt an diesem Ort an. Sobald die Spitze 34 (herausstehend aus dem Endabschnitt 32) diesen Knochenbereich erreicht hat, entnimmt der Operateur den Dorn 30 durch Herausziehen, woraufhin dann die Drahtelektrode 12 durch den nunmehr hohlen Hülsen-Innenraum des Werkzeugs 28, entlang der vorgegebenen Krümmung des Abschnitts 26, eingeführt und bis zum Kontaktbereich mit dem Gehäuse 10 gebracht werden kann. Das nachfolgende Herausziehen des Werkzeugs 28 (entgegen der Pfeilrichtung 36) bewirkt dann, dass die Drahtelektrode 12 bereits in der korrekten Anlageposition am Nerv anliegt, ohne dass die Gefahr von Verletzungen oder dergleichen Operationsfehlern bestehen kann.

Auch wenn das exemplarisch am Ausführungsbeispiel gezeigte System sich im besonderen Maße eignet, für die Neuromodulation von Nerven zur Sexualstimulation implantiert zu werden, ist die vorliegende Erfindung gleichwohl nicht auf diese Indikation beschränkt. Vielmehr eignet sich die vorliegende Erfindung für jegliche Indikation betreffend eine Neuromodulation bzw. Stimulation von Nerven, bei welchen einfache und sichere Implantierbarkeit mit bestmöglicher Befestigbarkeit und sicheren Betriebseigenschaften zu kombinieren sind.

## Patentansprüche

1. In einen menschlichen Körper implantierbares System zur Neuromodulation von Nerven mit
in einem implantierbaren Signalerzeugergehäuse (10) vorgesehenen elektronischen Signalerzeugungsmitteln,
mit den Signalerzeugungsmitteln verbundenen oder verbindbaren Spannungsversorgungsmitteln,
mindestens einer mit dem Signalerzeugergehäuse verbundenen oder verbindbaren, langgestreckt und zur Implantation an einem bevorzugt im Beckenboden und/oder Bauchbereich befindlichen Nerv (DNP) im menschlichen Körper ausgebildeten mehrkanaligen Drahtelektrodenmitteln (12),
die an einem Nervenkontaktbereich (14) eine Mehrzahl von Mantelsegmentelektroden (16a - 16d) ausbilden und endseitig mit Fixiermitteln (18) zum Verankern der Drahtelektrodenmittel an einem Implantationsort versehen sind, wobei das Signalerzeugergehäuse einen flanschartig und/oder gewebestückartig ansitzenden und/oder vorspringenden, flachen Befestigungsabschnitt (20) aufweist, der das operative Festlegen des Signalerzeugergehäuses an einem Gewebe-, Muskel- oder Knochenbereich (PB) im Körper, insbesondere an einem Bereich des Schambeins, an einem Muskelabschnitt und/oder an einer Faszie, ermöglicht und hülsenartige, gebogene und insbesondere über einen Betätigungsabschnitt (24) manuell betätigbare Elektrodendrahtführungsmittel (28), die von extrakorporal an einen Implantationsbereich zum Durchdringen von Gewebe des menschlichen Körpers
und zum Führen der Drahtelektrodenmittel (12) in einem hülsenförmigen Führungsabschnitt (26) der Elektrodendrahtführungsmittel sowie insbesondere zum Einführen der Drahtelektrodenmittel in den Führungsabschnitt von Intrakorporal, ausgebildet sind aufweist, **dadurch gekennzeichnet,**
**dass** die Elektrodendrahtführungsmittel einen in den hülsenförmigen Führungsabschnitt einsetzbaren und in einem in den Körper eingeführten Zustand der Elektrodendrahtführungsmittel von diesen durch Herausziehen aus einem körperseitigen Endbereich der Elektrodendrahtführungsmittel lösbaren Dorn (30) zugeordnet aufweist, der bevorzugt eine das Durchdringen ermöglichende Spitze (34) ausbildet.

2. System nach Anspruch 1, **dadurch gekennzeichnet, dass** der Befestigungsabschnitt elastisch verformbar ausgebildet und mit bevorzugt in Form eines regelmäßigen Musters angeordneten Durchbrüchen und/oder als mit Festlegungsmitteln in Form von Nahtmaterial, Schrauben, Nägeln oder Klammern durchdringbares Vlies, Polymermaterial, Netz und/oder Gewebe ausgebildet ist.

3. System nach Anspruch 1 oder 2, **dadurch gekennzeichnet, dass** der Befestigungsabschnitt (20) axial beidends von Schmalseiten eines bevorzugt langgestreckt ausgebildeten Signalerzeugergehäuses vorspringt.

4. System nach einem der Ansprüche 1 bis 3, **dadurch gekennzeichnet, dass** die Fixiermittel der Drahtelektrodenmittel als eine Fixation mittels Nahtmaterial erlaubende Öse (18) am freien Ende der Drahtelektrodenmittel (12) ausgebildet sind.

5. System nach einem der Ansprüche 1 bis 4, **dadurch gekennzeichnet, dass** das Signalerzeugergehäuse zum Verbinden mit den zumindest teilweise gehäuseextern in einem bevorzugt implantierbar ausgebildeten Spannungsversorgungsgehäuse vorgesehenen Spannungserzeugungsmitteln eine weiter bevorzugt lösbare Stecker- oder Buchsenanordnung aufweist.

6. System nach Anspruch 5, **dadurch gekennzeichnet, dass** das Spannungsversorgungsgehäuse über ein flexibles und zur Implantation vorgesehenes Spannungsversorgungskabel mit der Stecker- bzw. Buchsenverbindung kontaktierbar ausgebildet ist.

7. System nach einem der Ansprüche 1 bis 6, **dadurch gekennzeichnet, dass** die Spannungserzeugungsmittel eine bevorzugt induktiv und weiter bevorzugt von extrakorporal aufladbar ausgebildete Batterie aufweisen.

8. System nach einem der Ansprüche 1 bis 7, **dadurch gekennzeichnet, dass** die Spannungserzeugungsmittel implantierbare, mit Körpertemperatur thermisch wirksame Spannungserzeugungsmittel aufweisen oder mit diesen verbunden oder verbindbar sind.

9. System nach einem der Ansprüche 1 bis 8, **dadurch gekennzeichnet, dass** das Signalerzeugergehäuse zum gemeinsamen, bevorzugt lösbaren Anschließen einer Mehrzahl von Mehrkanaldrahtelektroden zur Realisierung der Drahtelektrodenmittel ausgebildet ist.

10. System nach einem der Ansprüche 1 bis 9, **gekennzeichnet durch** zum drahtlosen Kommunizieren mit den Signalerzeugungsmitteln in einem implantierten Zustand des Signalerzeugergehäuses ausgebildeten und zur extrakorporalen Bedienung ausgebildeten Steuer-, Parametrisierungs- und/oder Betriebsmodusmitteln,
die weiter bevorzugt zum mobilen oder portablen Betrieb und weiter bevorzugt zur manuellen Handhabung ausgebildet sind.

11. System nach Anspruch 10, **dadurch gekennzeichnet, dass** die Steuer-, Parametrisierungs- bzw. Betriebsmodusmitteln zur nutzer- und/oder geräteidentifizierenden Kommunikation mit den Signalerzeugungsmitteln ausgebildet sind
und/oder Mittel zur akustischen und/oder optischen Darstellung, insbesondere zur Visualisierung auf einer Displayeinheit, von einem Konfigurtions-, Stromversorgungs- und/oder Betriebszustand der Signalerzeugungsmittel aufweisen.

12. System nach einem der Ansprüche 1 bis 8, 10, 11, **dadurch gekennzeichnet, dass** die Drahtelektrodenmittel unlösbar mit dem Signalerzeugergehäuse verbunden sind
und/oder die im Signalerzeugergehäuse vorgesehenen Signalerzeugungsmittel zum Erzeugen eines einzelnen vorbestimmten Signalerzeugungsmusters für die Drahtelektrodenmittel konfiguriert sind, wobei das Signalerzeugungsmuster an eine Anzahl und/oder Ausrichtung von Mantelelektrodensegmenten der Drahtelektrodenmittel angepasst ist.

13. System nach einem der Ansprüche 1 bis 12, aufweisend eine Mehrzahl der implantierbaren Signalerzeugergehäuse mit jeweils darin vorgesehenen Signalerzeugungsmitteln, wobei die Signalerzeugergehäuse in einem in einem gemeinsamen Körper implantierten Zustand mittels bevorzugt lösbar vorgesehenen drahtförmigen Verbindungsmitteln elektronisch und signalleitend und/oder versorgungsspannungsführend miteinander verbunden und/oder verbindbar sind.

## Claims

1. A system for neuromodulation of nerves implantable into a human body and having electronic signal generating means provided in an implantable signal generator housing (10),
the system also having voltage supply means connected or connectable to the signal generating means,
at least one oblong multi-channel wire electrode means (12) connected or connectable to the signal generator housing and realized for implantation on a nerve (DNP), preferably in the pelvic floor and/or abdominal area, in the human body,
said multi-channel wire electrode means (12) realizing a plurality of shell segment electrodes (16a - 16d) on a nerve contact area (14) and, at the ends thereof, the multi-channel wire electrode means (12) being provided with fixing means (18) for anchoring the wire electrode means to an implantation location, the signal generator housing having a flat fixing section (20) which sits and/or protrudes in the manner of a flange and/or a piece of tissue and which allows the surgical fixation of the signal generator housing to a tissue, muscle or bone area (PB) in the body, in particular to an area of the pubic bone, to a muscle portion and/or to a fascia and the signal generator housing having sleeve-like, curved electrode wire guide means (28) which can be manually operated, in particular via an operation section (24), and which are realized for penetrating tissue of the human body from the extracorporeal at an implantation area
and for guiding the wire electrode means (12) in a sleeve-shaped guide section (26) of the electrode wire guide means and, in particular, for inserting the wire electrode means into the guide section from the intracorporeal,
**characterized in that**
the electrode wire guide means have an assigned mandrel (30) which can be inserted into the sleeve-shaped guide section and which can be detached from the electrode wire guide means by pulling out of an end portion on the body side of the electrode wire guide means when the electrode wire guide means are inserted into the body and which preferably realizes a tip (34) which allows the penetration.

2. The system according to claim 1, **characterized in that** the fixing section is realized so as to be elastically deformable and has openings which are preferably disposed in the form of a regular shaped pattern and/or is realized as a nonwoven fabric, polymer material, mesh and/or tissue which can be penetrated by fixing means in the form of suture material, screws, nails or clamps.

3. The system according to claim 1 or 2, **characterized in that** the fixing section (20) axially protrudes on both ends from narrow sides of a preferably oblong signal generator housing.

4. The system according to any one of claims 1 to 3, **characterized in that** the fixing means of the wire electrode means are realized as a grommet (18) which allows a fixation by means of suture material on the free end of the wire electrode means (12).

5. The system according to any one of claims 1 to 4, **characterized in that** the signal generator housing has a preferably detachable plug or socket assembly for the connection to the voltage generating means which are, at least partially, provided outside the housing in a preferably implantable voltage supply housing.

6. The system according to claim 5, **characterized in that** the voltage supply housing is realized so as to be contactable by the plug or socket connection via a flexible voltage supply cable which is provided for implantation.

7. The system according to any one of claims 1 to 6, **characterized in that** the voltage generating means have a battery which is realized so as to be chargeable in a preferably inductive manner and more preferably from the extracorporeal.

8. The system according to any one of claims 1 to 7, **characterized in that** the voltage generating means have implantable voltage generating means which are thermally effective at body temperature or are connected or can be connected to said voltage generating means.

9. The system according to any one of claims 1 to 8, **characterized in that** the signal generator housing is realized for the common, preferably detachable, connection of a plurality of multi-channel wire electrodes for realizing the wire electrode means.

10. The system according to any one of claims 1 to 9, **characterized by** control, parameterizing and/or operating mode means realized for the wireless communication with the signal generating means in an implanted state of the signal generator housing and realized for the extracorporeal operation,
said control, parameterizing and/or operating mode means being more preferably realized for the mobile or portable operation and, more preferably, for the manual handling.

11. The system according to claim 10, **characterized in that** the control, parameterizing or operating mode means are realized for the user-identifying and/or device-identifying communication with the signal generating means and/or that the control, parameterizing or operating mode means have means for the acoustic and/or optical representation, in particular for the visualization on a display unit, of a configuration, current supply and/or operating mode of the signal generating means.

12. The system according to any one of claims 1 to 8, 10, 11, **characterized in that** the wire electrode means are connected in an undetachable manner to the signal generator housing
and/or that the signal generating means provided in the signal generator housing are configured for generating a single predefined signal generating pattern for the wire electrode means, the signal generating pattern being adapted to a number and/or orientation of shell electrode segments of the wire electrode means.

13. The system according to any one of claims 1 to 12 comprising a plurality of the implantable signal generator housings having signal generating means provided therein, the signal generator housings being connected and/or connectable to one another in an electronic and signal-conducting manner and/or carrying the supply voltage by means of preferably detachable wire-shaped connecting means in an implanted state in a common body.

## Revendications

1. Système de neuromodulation des nerfs implantable dans un corps humain et comprenant
des moyens de génération de signal électroniques qui sont prévus dans un boîtier de générateur de signal (10) implantable,
des moyens d'alimentation en tension qui sont reliés ou peuvent être reliés aux moyens de génération de signal,
au moins un moyen de fil-électrode (12) multicanal allongé qui est relié ou peut être relié au boîtier de générateur de signal et qui est conçu pour l'implantation sur un nerf (DNP), de préférence dans le plancher pelvien et/ou dans la zone de l'abdomen, dans le corps humain,
lesdits moyens de fil-électrode (12) formant une pluralité d'électrodes de segment de gaine (16a - 16d) sur une zone de contact de nerf (14) et étant pourvus, du côté d'extrémité, de moyens de fixation (18) pour l'ancrage des moyens de fil-électrode sur un site d'implantation, dans lequel le boîtier de générateur de signal a une partie de fixation (20) plate qui siège et/ou fait saillie en forme de bride et/ou en forme d'une pièce de tissu et qui permet la fixation chirurgicale du boîtier de générateur de signal à une zone de tissu, de muscle ou d'os (PB) dans le corps, notamment à une zone du pubis, à une partie de muscle et/ou à un fascia, et qui a des moyens de guidage de fil d'électrode (28) courbés en forme de douille qui peuvent être actionnés manuellement, notamment par une partie d'actionnement (24), et qui sont conçus pour la pénétration dans de tissu du corps humain du côté extracorporel à une zone d'implantation
et pour le guidage des moyens de fil-électrode (12) dans une partie de guidage (26) en forme de douille des moyens de guidage de fil d'électrode et notamment pour l'insertion des moyens de fil-électrode du côté intracorporel dans la partie de guidage,
**caractérisé en ce que**
les moyens de guidage de fil d'électrode ont un mandrin (30) associé qui peut être inséré dans la partie de guidage en forme de douille et qui peut être détaché des moyens de guidage de fil d'électrode par le retrait d'une zone d'extrémité du côté de corps des moyens de guidage de fil d'électrode dans un état dans lequel les moyens de guidage de fil d'électrode sont insérés dans le corps, le mandrin (30) formant, de préférence, une pointe (34) qui permet la pénétration.

2. Système selon la revendication 1, **caractérisé en ce que** la partie de fixation est élastiquement déformable et a des ouvertures disposées, de préférence, d'une manière régulière et/ou que la partie de fixation est réalisée comme non-tissé, matériau polymère, maillage et/ou tissu qui peut être pénétré par des moyens de fixation en forme de matériau de suture, de vis, de clous ou d'agrafes.

3. Système selon la revendication 1 ou la revendication 2, **caractérisé en ce que** la partie de fixation (20) fait saillie aux deux extrémités axiales des côtés étroits d'un boîtier de générateur de signal, de préférence allongé.

4. Système selon l'une quelconque des revendications 1 à 3, **caractérisé en ce que** les moyens de fixation des moyens de fil-électrode sont réalisés comme œillet (18) qui permet une fixation au moyen d'un matériau de suture à l'extrémité libre des moyens de fil-électrode (12).

5. Système selon l'une quelconque des revendications 1 à 4, **caractérisé en ce que** le boîtier de générateur de signal a un ensemble de fiches ou de connecteurs, de préférence détachable, pour le raccordement aux moyens de génération de tension qui sont au moins partiellement prévus à l'extérieur du boîtier dans un boîtier d'alimentation en tension, de préférence implantable.

6. Système selon la revendication 5, **caractérisé en ce que** le boîtier d'alimentation en tension peut être mis en contact avec le raccordement de fiches ou de connecteurs par un câble d'alimentation en tension flexible qui est conçu pour l'implantation.

7. Système selon l'une quelconque des revendications 1 à 6, **caractérisé en ce que** les moyens de génération de tension ont une batterie qui peut être rechargée, de préférence de manière inductive, plus préférentiellement du côté extracorporel.

8. Système selon l'une quelconque des revendications 1 à 7, **caractérisé en ce que** les moyens de génération de tension ont ou sont reliés à ou peuvent être reliés à des moyens de génération de tension implantables qui sont thermiquement efficaces à la température du corps.

9. Système selon l'une quelconque des revendications 1 à 8, **caractérisé en ce que** le boîtier de générateur de signal est conçus pour la connexion commune, de préférence détachable, d'une pluralité de fil-électrodes multicanaux afin de réaliser les moyens de fil-électrode.

10. Système selon l'une quelconque des revendications 1 à 9, **caractérisé par** des moyens de commande, de paramétrage et/ou de mode de fonctionnement conçus pour la communication sans fil avec les moyens de génération de signal dans un état implanté du boîtier de générateur de signal et conçus pour la commande extracorporelle,
plus préférentiellement conçus pour le fonctionnement mobile ou portable et plus préférentiellement pour la manipulation manuelle.

11. Système selon la revendication 10, **caractérisé en ce que** les moyens de commande, de paramétrage ou de mode de fonctionnement conçus pour une communication avec les moyens de génération de signal qui permet d'identifier l'utilisateur et/ou le dispositif
et/ou que les moyens de commande, de paramétrage ou de mode de fonctionnement ont des moyens de représentation acoustique et/ou optique, notamment de visualisation sur une unité d'écran, d'un état de configuration, d'alimentation en courant et/ou de fonctionnement des moyens de génération de signal.

12. Système selon l'une quelconque des revendications 1 à 8, 10, 11, **caractérisé en ce que** les moyens de fil-électrode sont reliés de manière non-détachable au boîtier de générateur de signal
et/ou que les moyens de génération de signal prévus dans le boîtier de générateur de signal sont configurés pour générer un seul modèle de génération de signal pour les moyens de fil-électrode, dans lequel le modèle de génération de signal est adaptée à un nombre et/ou à une orientation de segments d'électrode de gaine des moyens de fil-électrode.

13. Système selon l'une quelconque des revendications 1 à 12 comprenant une pluralité de boîtiers de générateur de signal implantables, chaque boîtier de générateur de signal ayant des moyens de génération de signal prévus dans celui-ci, dans lequel, dans un état implanté dans un corps commun, les boîtiers de générateur de signal sont reliés et/ou peuvent être reliés l'un à l'autre de manière électronique et de manière à conduire le signal et/ou de manière à conduire la tension d'alimentation au moyen de moyens de raccordement en forme de fil, de préférence prévus de manière détachable.
